# EUROPÄISCHE PATENTSCHRIFT

(11) **EP 0 993 319 B1**
(45) Veröffentlichungstag und Bekanntmachung des Hinweises auf die Patenterteilung: **13.03.2002**
(21) Anmeldenummer: 99929053.9
(22) Anmeldetag: 30.04.1999
(51) Int. Cl.: A61N 5/10

(54) **KATHETER ODER GEFÄSSSTÜTZE MIT STRAHLENSENSIBILISATOR UND VERFAHREN ZUR HERSTELLUNG**
CATHETER OR VESSEL SUPPORT WITH RADIATION SENSITIZER AND CORRESPONDING PRODUCTION METHOD
CATHETER OU TUTEUR VASCULAIRE AVEC SENSIBILISATEUR AUX RAYONNEMENTS, ET SON PROCEDE DE PRODUCTION

(30) Priorität: 30.04.1998 DE 19819426
(43) Veröffentlichungstag der Anmeldung: 19.04.2000
(73) Patentinhaber: Hehrlein, Christoph, 69118 Heidelberg (DE)
(72) Erfinder: HEHRLEIN, Christoph, D-69118 Heidelberg (DE); WOLF, Gerhard, Karl, D-69120 Heidelberg (DE)
(74) Vertreter: KOHLER SCHMID + PARTNER
(86) Internationale Anmeldenummer: DE9901267
(87) Internationale Veröffentlichungsnummer: WO9956828

(56) Entgegenhaltungen:
- WO-A-93/04735
- DE-A- 19 600 669
- US-A- 4 727 068
- US-A- 4 742 050
- US-A- 5 102 402
- US-A- 5 674 192

## Beschreibung

Die vorliegende Erfindung betrifft einen Katheter oder Gefäßstütze, insbesondere zur Verhinderung der Re-Stenose nach Angioplastie, mit einer auf zu behandelndes Gewebe lokal einwirkenden radioaktiven Strahlungsquelle, sowie Verfahren zu ihrer Herstellung.

Der Katheter oder die Gefäßstütze dienen zur Verhinderung der Restenose nach Angioplastie.

Ein radioaktiver Ballon-Katheter zur Verhinderung der Re-Stenose nach Angioplastie ist zum Beispiel durch die **WO-A-9 304 735** bekanntgeworden.

Die Angioplastie durch einen Ballon-Katheter zur Behandlung von symptomatischen Gefäßeinengungen (Stenosen) der Koronararterien ist eine Therapieform, bei der es bei 30-50% der behandelten Patienten zu einer Re-Stenose und damit zum erneut drohenden Herzinfarkt kommt. Auch andere Methoden der Angioplastie durch Laser, die Atherektomie (Ausschälung von arteriosklerotischen Plaques) und die Implantation von Gefäßstützen (Stents) können eine Re-Stenose herbeiführen. Unterschiedliche Ansätze zur Verhinderung der Re-Stenose sind bekannt. Das Spektrum reicht von Medikamenten, die das überschießende Zellwachstum nach Angioplastie hemmen bis hin zur Strahlentherapie, die ebenfalls eine Reduktion der Gewebswucherung hervorruft.

Ein grundsätzliches Ziel bei der Herstellung aller radioaktiven Instrumente für die Strahlentherapie in der Medizin für sogenannte Brachytherapie ist es, die Menge an Radioaktivität, die notwendig ist, um eine therapeutische Wirkung durch ionisierende Strahlung zu erzielen, so gering wie möglich zu halten. Herkömmliche radioaktive Katheter enthalten je nach Strahlenart (Beta- öder Gammastrahlung) radioaktive Partikel mit einer Aktivität zwischen 1 mCi und 10 Ci. Es ist bekannt, daß der anti-proliferative Effekt der Strahlentherapie durch eine wachstumshemmende Medikamentenbehandlung verstärkt bzw. verbessert werden kann und daß Medikamente das Gewebe für die Therapie mit radioaktiver Strahlung sensibilisieren können.

Aus der WO-A-9 304 735 ist es bekannt, dass die radioaktive Wirkung durch Strahlensensibilisatoren gesteigert werden kann. Diese Mittel können entweder oral, injektiv oder rektal oder aber lokal verabreicht werden. Im letzten Fall wird der Strahlensensibilisator durch einen perforierten Ballon-katheter verabreicht. Da alle in der WO-A-9 304 735 gezeigten Katheter radioaktive Elemente tragen, aber nicht perforiert sind, erfolgt die lokale Verabreichung des Strahlensensibilisators offensichtlich zunächst mittels eines ersten perforierten Ballonkatheters und erst danach wird ein zweiter radioaktiver Ballonkatheter an das nun sensibilisierte Gewebe herangeführt.

Aus der **DE 196 00 669** ist weiterhin ein Katheter mit einer homogen radioaktiv veränderten Ballonwand bekannt. Eine homogene Bestrahlung im Nahbereich wird durch die Art der Herstellung gewährleistet, die entweder in Form einer direkten Implantation von Radioisotopen in die Ballonwand oder durch eine Beschichtung der Ballonwand mit einem Film, der Radioisotope enthält, oder durch Zumischen von Radioisotopen bei der Ballonherstellung erreicht wird. Diese homogene Verteilung der Radioaktivität an bzw. in der Ballonwand ist für die Verhinderung der Re-Stenose durch Strahlentherapie von besonderem Vorteil.

Der vorliegenden Erfindung liegt die Aufgabe zugrunde, einen Katheter und eine Gefäßstütze der eingangs genannten Art derart weiterzubilden, dass das zu behandelnde Gewebe mit möglichst geringem Aufwand für die radioaktive Therapie sensibilisiert werden kann, sowie ein Verfahren zu ihrer Herstelllung anzugeben.

Die Aufgabe wird erfindungsgemäß dadurch gelöst, dass ein das zu behandelnde Gewebe für die radioaktive Therapie durch Reduktion der Gewebshypoxie sensibilisierender Strahlensensibilisator im radioaktiven Wirkbereich an oder in der Außenwand bzw. Außenoberfläche des Katheters oder der Gefäßstütze vorgesehen ist und nach außen an das Gewebe abgebbar ist. Der Strahlensensibilisator ist entweder gasförmig oder flüssig. Anwendbare Substanzen sind z.B. reiner Sauerstoff, Stickoxid, Sauerstoffträger oder Metallionenkomplexe.

Erfindungsgemäß wird in einem Instrument die lokale, radioaktive Strahlentherapie mit der lokalen Gabe einer Substanz, die Gewebshypoxie reduziert, kombiniert. Der Vorteil eines solchen Katheters bzw. Gefäßstütze zur kombinierten, radioaktiven Strahlen- und Hypoxietherapie besteht darin, daß eine geringere Radioaktivität des Katheters notwendig ist, um die gleiche Wirkung wie ein herkömmlicher radioaktiver Katheter zu erzielen. Dies trägt zu einer ökonomischeren Herstellung von Kathetern mit einem radioaktivem Element bei. Das Gewebe wird erst mit einem Strahlensensibilisator versehen und dann bestrahlt.

Vorzugsweise ist die Katheter-Außenwand bzw. -Außenoberfläche oder die Ballonwand schwammartig oder porös zur Einlagerung des Strahlensensibilisators in fester, flüssiger oder gasförmiger Form ausgebildet. In Kontakt mit Gewebe oder durch Expansion des Ballons wird z.B. der Strahlensensibilisator dann freigesetzt bzw. gelöst. Alternativ kann der Strahlensensibilisator auch durch Löcher im Katheter oder durch eine poröse Ballonmembran direkt in das Gefäß injiziert werden.

Bei ganz besonders bevorzugten Ausführungsformen der Erfindung, weist die Außenwand bzw. Außenoberfläche oder Ballonwand mehrere Schichten, insbesondere Polymerschichten (Polymerfilm) auf, von denen mindestens eine Schicht radioaktiv ist und mindestens eine andere Schicht den Sensibilisator aufweist. Für ein Recyclen von radioaktivem Material sind die einzelnen Schichten in, insbesondere organischen, Lösungsmittel, wie z.B. Chloroform oder Alkohol, selektiv oder sukzessiv entfernbar. Zwei benachbarte Schichten können durch Adhäsionskräfte miteinander verankert sein. Möglich ist z.B. ein Schichtenaufbau mit einer nichtradioaktiven inneren Schicht, mit einer die mindestens eine Radionuklidspecies enthaltenden mittleren Schicht, die auf der inneren Schicht aufgebracht ist, und einer Schutzschicht, die auf der mittleren Schicht aufgebracht ist, wobei bereits diese Schutzschicht den Sensibilisator enthalten kann. Oder auf der Schutzschicht ist noch eine den Sensibilisator aufweisende äußere Schicht vorgesehen.

In einer anderen vorteilhaften Ausführungsform löst sich die den Sensibilisator aufweisende äußere Schicht bei Erwärmung auf. Diese Auflösung kann insbesondere bei Erwärmung aufgrund der Körpertemperatur oder am Katheter vorgesehener externer Heizmittel erfolgen. Die Schicht (Polymerschicht) löst sich, ohne selbst komprimiert zu werden, temperaturabhängig vollständig auf und gibt den Strahlensensibilisator zur Diffusion in das Gewebe frei.

Die Schichten können aus Zellulose oder deren Abkömmlingen, Polyäthylen, Polypropylen, Polypyrrol, Polythiopen oder Nylon gebildet sein. Insbesondere sind thermoplastische Kunststoffe geeignet.

Vorzugsweise werden als Radionuklide Alpha- Beta- oder Gamma-Strahler, vorzugsweise P-32, Y-90, Re-188, Pd-103, Ir-192, Xe-133, 0-15 oder Tc-99m, eingesetzt. Mit einem Schutz (Schutzhülle), der während des Gebrauchs des Katheters, Ballon-Katheters oder der Gefäßstütze zurückgezogen ist, läßt sich die radioaktive Strahlung abschirmen.

Die Erfindung betrifft auch Verfahren zum Herstellen solcher Katheter, Ballon-Katheter oder Gefäßstützen, wobei erfindungsgemäß vorgeschlagen wird, diese radioaktiven Instrumente mit dem Strahlensensibilisator zu beschichten.

In bevorzugter Verfahrensausgestaltung wird der radioaktive Katheter, Ballon-Katheter oder die radioaktive Gefäßstütze mit einem Polymer oder mit Polymeren beschichtet, das bzw. die den nach außen abgebbaren Strahlensensibilisator enthalten. Dabei können insbesondere Polyacrylsäure und deren Abkömmlinge oder Polyvinylalkohol und dessen Abkömmlinge, zur Herstellung der den Sensibilisator enthaltenden Polymerbeschichtung verwendet werden. Am einfachsten kann die Beschichtung durch Eintauchen in das entsprechende Polymer oder in eine entsprechende Polymerlösung auf die Außenwand bzw. Außenoberfläche des Katheters oder der Gefäßstütze oder auf die Ballonwand des Ballon-Katheters aufgebracht werden.

Vorteilhaft ist bei einem Ballonkatheter der Strahlensensibilisator durch die Ballonwand von der Radionuklidspecies getrennt. Dazu kann die Innenoberfläche des Ballons mit einer die Radionuklidspecies enthaltenden Polymerlösung beschichtet und auf die Außenoberfläche der Strahlensensibilisator aufgebracht werden. Zum Beispiel kann die auf die Innenoberfläche des Ballons aufgebrachte Polymerschicht anschließend zur Polymerisation gebracht werden.

Zur Herstellung solcher Katheter wird z.B. ein Verfahren zur Beschichtung von Kathetern mit Polymeren vorgechlagen, die den Strahlensensibilisator, z.B. reinen, molekularen Sauerstoff oder einen Sauerstoffträger, in flüssiger oder gasförmiger Form enthalten. Zur Anwendung dieses Herstellungsverfahrens eignen sich prinzipiell alle Katheter oder Gefäßstützen (röhrenförmig oder gitterförmig), die in Kontakt mit dem zu bestrahlenden Gewebe gebracht werden. Eine homogene Verteilung der Radioaktivität kann mit gitterförmigen Gefäßstützen nicht erreicht werden. Im unten genannten Beispiel 1 wird diese Beschichtung für einen Ballon-Katheter beschrieben. Die Beschichtung wird dadurch erreicht, daß zunächst ein radioaktiver Ballon-Katheter hergestellt und anschließend mit einer schwammartigen, hydrophilen oder lipophilen Polymerbeschichtung an der Außenwand des Ballons versehen wird, die mit dem Strahlensensibilisator in Lösung beladen ist. Während der Aufdehnung des Ballons wird der Strahlensensibilisator durch Kompression der Polymerschicht in die Gefäßwand gepreßt. Die Substanz wirkt gleichzeitig mit der radioaktiven Strahlung auf die Gefäßwand ein. Alternativ wird ein radioaktives Kathetersystem oder ein Stent mit einer Polymerbeschichtung versehen, die in Kontakt mit dem erkrankten Gewebe gebracht wird. Das Polymer löst sich, ohne selbst komprimiert zu werden, temperaturabhängig vollständig auf und gibt die Substanz zur Diffusion in das Gewebe frei. Als weitere Alternative ist der Ballonkatheter porös und der Strahlensensibilisator wird mechanisch durch die Löcher injiziert. Der Vorteil eines Katheters zur kombinierten, radioaktiven Strahlen- und Sensibilisierungstherapie ist, daß eine geringere Radioaktivität des Katheters notwendig ist, um die gleiche Wirkung wie ein herkömmlicher radioaktiver Katheter zu erzielen. Dies trägt zu einer ökonomischeren Herstellung von Kathetern mit radioaktiven Elementen bei. Ein Katheter kann dann noch mit einer strahlenundurchlässigen Schiene bzw. Schützhülle aus Kunststoff oder flexiblem Metall versehen werden, die vor Gebrauch zurückgezogen wird. Die genannten Herstellungsverfahren eines radioaktiven Katheters zur Strahlentherapie beziehen sich auf die Verwendung von Kunststoffen, die in Form mehrerer Schichten aufgrund von Adhäsionskräften an der Ballonwand fixiert werden. Diese Schichten enthalten Radioisotope und Substanzen, die in unterschiedlichen Polymeren bzw. Polymerschichten lagern. Im folgenden werden mögliche Herstellungsverfahren anhand von Beispielen beschrieben.

### Beispiel 1: Polymerbeschichtung des radioaktiven Ballons, die Substanzen zur Hypoxietherapie enthält

Jeder bekannte Prozeß kann dazu verwendet werden, einen Ballon-Katheter zur radioaktiven Strahlentherapie herzustellen.

Das Prinzip des radioaktiven Ballon-Katheters mit Strahlensensibilisator ist es, eine gleichzeitige radioaktive Bestrahlung und Hypoxietherapie in der Gefäßwand zu erreichen. Gase wie Sauerstoff oder Stickoxid werden in einem wasseraufnehmenden Polymer (z.B. Polyacrylsäure und deren Abkömmlinge bzw. Co-Polymere) gelöst. Alternativ wird ein flüssiger Sauerstoff- oder Stickoxidträger mit Polyvinylalkohol und dessen Abkömmlinge vermengt, einen Polymer, welches durch Wasser zersetzt werden kann. Diese medikamentenhaltige Polymerlösung wird im Tauchverfahren auf die Außenseite der radioaktiven Ballonwand aufgebracht, die wie oben bereits beschrieben, mit einer Schutzschicht versehen ist, welche die Vermischung von Radioisotopen und dem Strahlensensibilisator verhindert. Die den Strahlensensibilisator enthaltende Polymerverbindung auf der Ballonoberfläche wird bei der Aufdehnung des Ballons gegen die Gefäßwand komprimiert und/oder aufgelöst, so daß die Substanz in die Gefäßwand einwandern bzw. diffundieren kann. Alternativ werden zwei verschiedene Sauerstoffträger in diese Schicht eingebracht, welches die Strahlensensibilität des Gewebes weiter erhöht. Das Prinzip beruht auf dem direkten Kontakt dieses Katheters mit dem erkrankten Gewebe, z.B. einer stenosierten Koronararterie. Der Strahlensensibilisator kann entweder durch Kompression des Polymers gegen das Gewebe oder durch temperaturabhängiges Auflösen der Polymerschicht an das erkrankte Gewebe abgegeben werden.

### Beispiel 2: Innenbeschichtung des Ballon-Katheters mittels UV-Polymerisation

Ein geeignetes Monomer (z.B. Vinylazetat) oder ein nichtvernetztes Polymer (z.B. Polyäthylen) wird in Lösung gebracht, mit Radioisotopen versetzt und über einen dünnen Katheterschlauch in das Innere des Ballons gebracht. Die radioaktive Lösung wird durch UV-Strahlung oder Temperaturerhöhung polymerisiert bzw. vernetzt. Eine gleichmäßige Innenwandbelegung wird durch Rotation des Ballons während der Polymerisation erreicht. Der radioaktive Polymerfilm haftet fest in der Innenseite des Ballons. Der Strahlensensibilisator ist an der Außenwand des Ballons angebracht und wird beim Aufdehnen des Ballons in das Zielgewebe befördert. Alternativ ist die Außenfläche des Ballons porös und mit einem Injektionskanal im Katheter verbunden. Die Außenfläche ist schlauchförmig konfiguriert und läßt die Injektion eines flüssigen Sauerstoffträgers zu.

Weitere Vorteile der Erfindung ergeben sich aus der Beschreibung und der Zeichnung. Ebenso können die vorstehend genannten und die noch weiter aufgeführten Merkmale erfindungsgemäß jeweils einzeln für sich oder zu mehreren in beliebigen Kombinationen Verwendung finden. Die gezeigte und beschriebene Ausführungsform ist nicht als abschließende Aufzählung zu verstehen, sondern hat vielmehr beispielhaften Charakter für die Schilderung der Erfindung.

Es zeigt:

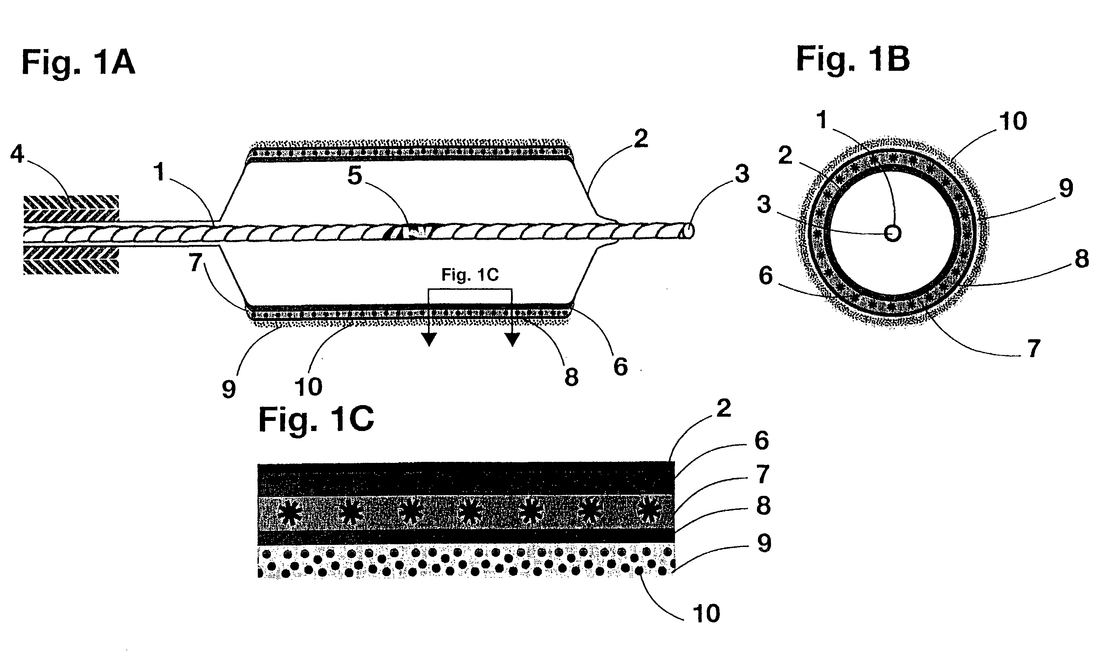

- Fig. 1: schematisch einen erfindungsgemäßen radioaktiven Ballon-Katheter in einer Längsansicht (Fig. 1a), einer Querschnittsansicht (Fig. 1b) und einer Detailansicht (Fig. 1c) ; und
- Fig. 2: den Ballon-Katheter der Fig. 1, der zur Behandlung in ein Gefäß eingeführt worden ist, zu einem späteren Behandlungszeitpunkt.

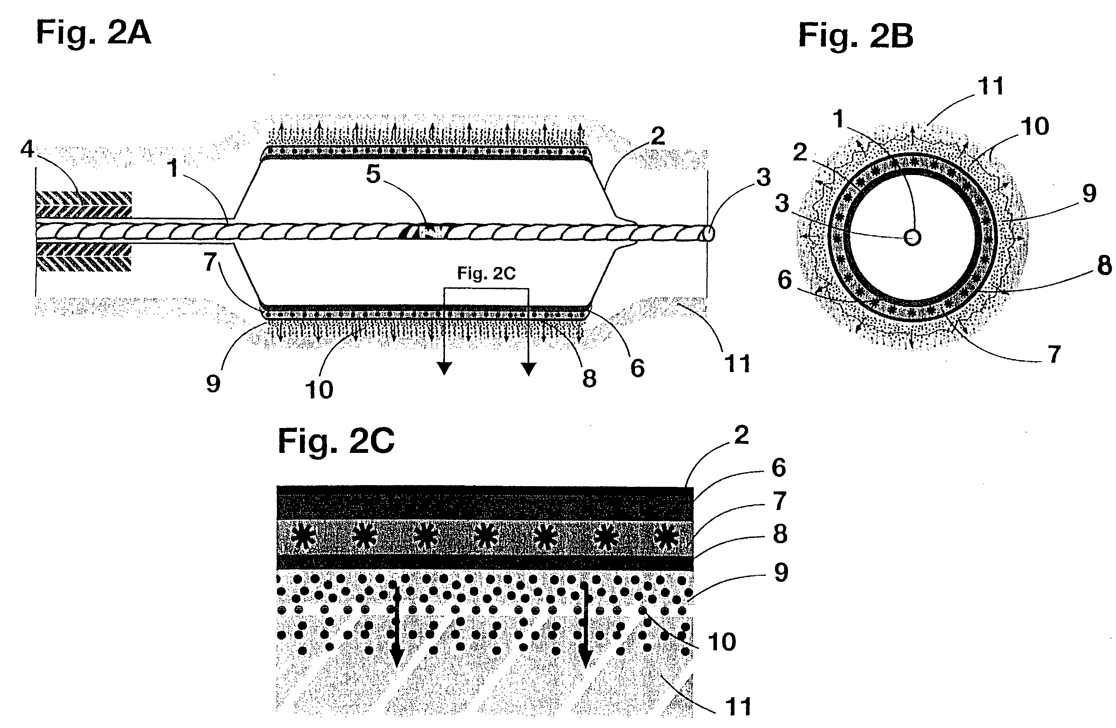

Der in der Zeichnung dargestellte Ballon-Katheter besteht aus einem Katheter **1**, an dessen Spitze sich ein Ballon **2** befindet. Durch das Lumen **3** des Katheters wird ein Führungsdraht in das Gefäß **11** (Fig. 2) geschoben. Um den Ballon-Katheter kann sich eine strahlenundurchlässige, flexible Schutzhülle **4** aus Kunststoff oder flexiblem Metall befinden, die vor der Aufdehnung des Ballons 2 zurückgezogen wird. Sie dient dazu, Strahlung und Medikation nicht während des Einführens und des Vorschiebens des Ballon-Katheters abzugeben, sondern nur am Wirkort in dem Gefäß (Arterie) 11. Nachdem die Schutzhülle 4 zurückgezogen wurde, wird der Ballon 2 im Gefäß 11 aufgedehnt. Es befindet sich eine röntgendichte Markierung **5** am Ballon-Katheter, welche die Mitte des Ballons 2 unter Röntgendurchleuchtung anzeigt. Der Ballon 2 weist eine dünne, leitfähige Polymerschicht **6** auf (Beispiel 1) und darüber befindet sich eine radioaktive Polymerschicht **7**. Auf dieser Schicht 7 ist eine Schutzschicht **8** angebracht, welche den Verlust von radioaktiven Partikeln verhindert. Abschließend ist eine Polymerschicht **9** befestigt, die den Strahlensensibilisator **10** enthält (Beispiel 1). Durch Aufdehnung des Ballons 2 und seine Kompression an die Gefäßwand wird die äußere Polymerschicht 9 zusammengedrückt, und der Strahlensensibilisator 10 wird während der Gefäßbestrahlung durch die radioaktiven Partikel des Katheters in die Gefäßwand 11 abgegeben (Fig. 2).

Entscheidend ist, daß ein solcher Katheter oder eine Gefäßstütze durch den Strahlensensibilisator nicht zwingend selbst radioaktiv sein müssen; es kann eine radioaktive Bestrahlung des Zielbgewebes auch mit einem anderen Instrument oder perkutan vor oder nach der Abgabe des Strahlensensibilisators erfolgen. Der erfindungsgemäße Vorteil gegenüber dem Stand der Technik beruht auf folgenden Aspekten:
1. Die Gabe eines Strahlensensibilisators erfolgt über den Katheter oder die Gefäßstützte lokal in die Gefäßwand zur Verbesserung der Effekte einer radioaktiven Bestrahlung der Gefäßwand.
2. Eine simultane Abgabe des Strahlensensibilisators an die Gefäßwand und die praktisch gleichzeitige ionisierende Bestrahlung wird als bevorzugte Version der Erfindung ausgewiesen.
3. Der Strahlensensibilisator soll die Hypoxie der Gefäßwand verhindern, welche nach einer Ballon-Angioplastie oder dem Einbau eines Stents auftritt.

Der Strahlensensibilisator ist im weitesten Sinne eine Substanz, die die Hypoxie der Gefäßwand reduziert bzw. vermindert. Der Strahlensensibilisator kann z.B. reiner Sauerstoff oder Stickoxid, ein Sauerstoffträger oder ein Metallionenkomplex sein. Die Substanzen zur Verminderung der Gefäßwandhypoxie können auch miteinander kombiniert werden.

Der Einsatz des Strahlensensibilisators im Rahmen dieser Erfindung ist insbesondere bei einer lokalen Anwendung radioaktiver Strahlung an der Gefäßwand sinnvoll, d.h. die Zielorte der Therapie müssen identisch sein.

Der Strahlensensibilisator kann auch über eine schlauchförmige poröse Ballonoberfläche direkt in die Gefäßwand injiziert werden.

## Patentansprüche

1. Katheter (1) oder Gefäßstütze, insbesondere zur Verhinderung der Re-Stenose nach Angioplastie, mit einer auf zu behandelndes Gewebe (11) lokal einwirkenden radioaktiven Strahlungsquelle (7),
**dadurch gekennzeichnet,**
**daß** ein das zu behandelnde Gewebe (11) für die radioaktive Therapie durch Reduktion der Gewebshypoxie sensibilisierender Strahlensensibilisator (10) im radioaktiven Wirkbereich an oder in der Außenwand bzw. Außenoberfläche des Katheters (1) oder der Gefäßstütze vorgesehen ist und nach außen an das Gewebe (11) abgebbar ist.

2. Katheter (1) nach Anspruch 1, **gekennzeichnet durch** einen Ballon (2), in oder an dessen Ballonwand der Strahlensensibilisator (10) vorgesehen ist.

3. Katheter oder Gefäßstütze nach Anspruch 1 oder 2, **dadurch gekennzeichnet, daß** der Strahlensensibilisator Sauerstoff, Stickoxid, ein Sauerstoffträger oder ein Metallionenkomplex ist.

4. Katheter oder Gefäßstütze nach einem der vorhergehenden Ansprüche, **dadurch gekennzeichnet, daß** die Außenwand bzw. -Außenoberfläche oder die Ballonwand schwammartig oder porös zur Einlagerung des Strahlensensibilisators (10) in fester, flüssiger oder gasförmiger Form ausgebildet ist.

5. Katheter oder Gefäßstütze nach einem der vorhergehenden Ansprüche, **dadurch gekennzeichnet, daß** die Außenwand bzw. Außenoberfläche oder Ballonwand mehrere Schichten, insbesondere Polymerschichten (6, 7, 8, 9) aufweist, von denen mindestens eine Schicht (7) radioaktiv -ist und mindestens eine andere Schicht (9) den Strahlensensibilisator (10) aufweist.

6. Katheter oder Gefäßstütze nach Anspruch 5, **gekennzeichnet durch** eine nichtradioaktive innere Schicht (6), eine mindestens eine Radionuklidspecies enthaltende mittlere Schicht (7), die auf der inneren Schicht (6) aufgebracht ist, und eine Schutzschicht (8), die auf der mittleren Schicht (7) aufgebracht ist.

7. Katheter oder Gefäßstütze nach einem der vorhergehenden Ansprüche, **dadurch gekennzeichnet, daß** Alpha- Beta- oder Gamma-Strahler, vorzugsweise P-32, Y-90, Re-188, Pd-103, Ir-192, Xe-133, 0-15 oder Tc-99m, als die mindestens eine Radionuklidspecies vorgesehen sind.

8. Katheter oder Gefäßstütze nach einem der vorhergehenden Ansprüche, **gekennzeichnet durch** einen die Außenwand (9) im radioaktiven Wirkbereich nach außen abschirmenden Schutz (4), der während des Gebrauchs des Katheters (1) oder der Gefäßstütze zurückgezogen ist.

9. Verfahren zum Herstellen eines Katheters (1) oder einer Gefäßstütze nach einem der vorhergehenden Ansprüche, **dadurch gekennzeichnet, daß** ein radioaktiver Katheter (1), Ballon-Katheter oder eine radioaktive Gefäßstütze mit dem Strahlensensibilisator (10) beschichtet wird.

10. Verfahren nach Anspruch 9, **dadurch gekennzeichnet, daß** die Oberfläche, insbesondere die Innenoberfläche, des Ballons (2) eines Ballon-Katheters mit einer den Strahlensensibilisator (10) enthaltenden Polymerlösung beschichtet wird, die anschließend zur Polymerisation gebracht wird.

## Claims

1. A catheter (1) or vascular brace, particularly for preventing re-stenosis following angioplasty with a radioactive radiation source (7) acting locally on tissue (11) which is to be treated, **characterised in that** a radiation sensitiser (10) which sensitises the tissue (11) to be treated for radioactive therapy by reduction of tissue hypoxia is provided in the radioactive effect region on or in the outer wall or outer surface of the catheter (1) or of the vascular brace and is adapted to pass outwards to the tissue (11).

2. A catheter (1) according to claim 1, **characterised by** a bulb (2) in or on the wall of which the radiation sensitiser (10) is provided.

3. A catheter or vascular brace according to claim 1 or 2, **characterised in that** the radiation sensitiser is oxygen, nitric oxide, an oxygen carrier or a metal ion complex.

4. A catheter or vascular brace according to one of the preceding claims, **characterised in that** the outside wall or outer surface or the bulb wall is sponge-like or porous for storing the radiation sensitiser (10) in solid, fluid or gaseous form.

5. A catheter or vascular brace according to one of the preceding claims, **characterised in that** the outer wall or outer surface or bulb wall comprises a plurality of layers, particularly layers (6, 7, 8, 9) of polymer, of which at least one layer (7) is radio-active and at least one other layer (9) comprises the radiation sensitiser (10).

6. A catheter or vascular brace according to claim 5, **characterised by** a non-radioactive inner layer (6), a middle layer (7) containing at least one radionuclide species and which is applied onto the inner layer (6), and a barrier layer (8) which is applied onto the middle layer (7).

7. A catheter or vascular brace according to one of the preceding claims, **characterised in that** alpha, beta or gamma ray radiators, preferably P-32, Y-90, Re-188, Pd-103, Ir-192, Xe-133, O-15 or Tc-99m are provided as the at least one radionuclide species.

8. A catheter or vascular brace according to one of the preceding claims, **characterised by** a barrier (4) which outwardly screens the outer wall (9) in the area of radio-active effect and which is retracted while the catheter (1) or vascular brace is in use.

9. A method of producing a catheter (1) or vascular brace according to one of the preceding claims, **characterised in that** a radioactive catheter (1), bulb catheter or a radioactive vascular brace is coated with the radiation sensitiser (10).

10. A method according to claim 9, **characterised in that** the surface, particularly the inner surface, of the bulb (2) of a bulb catheter is coated with a polymer solution containing the radiation sensitiser (10) and which is then brought to the polymerisation stage.

## Revendications

1. Cathéter (1) ou extenseur, en particulier pour éviter la resténose après une angioplastie, avec une source de rayon nement (7) radioactive agissant localement sur le tissu à traiter (11), **caractérisé en ce qu'**il est prévu sur ou dans la paroi externe ou la surface externe du cathéter (1) ou de l'extenseur un sensibilisateur aux rayonnements (10) sensibilisant le tissu à traiter (11) dans le domaine d'effet radioactif pour la thérapie radioactive par réduction de l'hypoxie tissulaire et qui peut être délivré à l'extérieur au tissu (11).

2. Cathéter (1) selon la revendication 1, **caractérisé par** un ballonnet (2) dans ou sur la paroi duquel est prévu le sensibilisateur aux rayonnements (10).

3. Cathéter ou extenseur selon la revendication 1 ou 2, **caractérisé en ce que** le sensibilisateur aux rayonnements est l'oxygène, le monoxyde d'azote, un porteur d'oxygène ou un complexe d'ions métalliques.

4. Cathéter ou extenseur selon l'une des revendications précédentes, **caractérisé en ce que** la paroi externe ou la surface externe ou la paroi du ballonnet est spongieuse ou poreuse pour l'inclusion du sensiblisateur aux rayonnements (10) sous forme solide, liquide ou gazeuse.

5. Cathéter ou extenseur selon l'une des revendications précédentes, **caractérisé en ce que** la paroi externe ou surface externe ou paroi du ballonnet comporte plusieurs couches, en particulier des couches de polymère (6, 7, 8 et 9), parmi lesquelles au moins une couche (7) est radioactive et au moins une autre couche (9) comporte le sensibilisateur aux rayonnements (10).

6. Cathéter ou extenseur selon la revendication 5, **caractérisé par** une couche interne non radioactive (6), une couche intermédiaire (7) contenant au moins une espèce de radionucléide, qui est appliquée sur la couche interne (6), et une couche protectrice (8) qui est appliquée sur la couche intermédiaire (7).

7. Cathéter ou extenseur selon l'une des revendications précédentes, **caractérisé en ce que** des émetteurs de rayonnement α, β ou γ, de préférence P-32, Y-90, Re-188, Pd-103, Ir-192, Xe-133, O-15 ou Tc-99m, sont prévus comme espèce(s) de radionucléide.

8. Cathéter ou extenseur selon l'une des revendications précédentes, **caractérisé par** une protection (4) isolant la paroi externe (9) de l'extérieur dans le domaine d'effet radioactif, qui est retirée pendant l'utilisation du cathéter (1) ou de l'extenseur.

9. Procédé de fabrication d'un cathéter (1) ou d'un extenseur selon l'une des revendications précédentes, **caractérisé en ce qu'**un cathéter radioactif (1), un cathéter à ballonnet ou un extenseur radioactif est recouvert d'un sensibilisateur aux rayonnements (10).

10. Procédé selon la revendication 9, **caractérisé en ce que** la surface, en particulier la surface interne, du ballonnet (2) d'un cathéter à ballonnet est recouverte d'une solution de polymère contenant le sensibilisateur aux rayonnements (10), qui est ensuite polymérisée.
